# FASCICULE DE BREVET EUROPEEN

(11) **EP 3 578 654 B1**
(45) Date de publication et mention de la délivrance du brevet: **09.04.2025**
(21) Numéro de dépôt: 19187302.5
(22) Date de dépôt: 27.01.2014
(51) Int. Cl.: C12N 15/10

(54) **PROCÉDÉ D'ISOLEMENT SPÉCIFIQUE D'ACIDES NUCLÉIQUES D'INTÉRÊT**
SPEZIFISCHES ISOLATIONSVERFAHREN VON NUCLEINSÄUREN VON INTERESSE
METHOD FOR SPECIFIC ISOLATION OF NUCLEIC ACIDS OF INTEREST

(30) Priorité: 25.01.2013 FR 1350650
(43) Date de publication de la demande: 11.12.2019
(62) Demande divisionnaire de: 14704612.2
(73) Titulaire: bioMérieux, 69280 Marcy l'Etoile (FR)
(72) Inventeur: TARENDEAU, Franck, 38100 Grenoble (FR)
(74) Mandataire: Atout PI Laplace

(56) Documents cités:
- EP-A2- 0 745 849
- WO-A1-2009/015484
- WO-A1-2010/062350
- BOUGNOUX M-E ET AL: "Serum is more suitable than whole blood for diagnosis of systemic candidiasis by nested PCR", JOURNAL OF CLINICAL MICROBIOLOGY, AMERICAN SOCIETY FOR MICROBIOLOGY, US, vol. 37, no. 4, 1 April 1999 (1999-04-01), pages 925 - 930, XP002210131, ISSN: 0095-1137
- CAMERON C M ET AL: "SEDIMENTATION OF BACTERIA WITH POLY ETHYLENE GLYCOL", 1974, ONDERSTEPOORT JOURNAL OF VETERINARY RESEARCH, VOL. 41, NR. 2, PAGE(S) 75-78, ISSN: 0030-2465, XP009515843

## Description

La présente invention concerne un procédé d'isolement sélectif de micro-organismes d'intérêt et/ou d'acides nucléiques d'intérêt au sein d'un échantillon biologique liquide contenant ou susceptible de contenir, notamment, de très nombreuses cellules non ciblées et/ou de très nombreux acides nucléiques de cellules non ciblées. Elle concerne également leur utilisation, des tests de diagnostic basés sur un procédé d'isolement de micro-organismes d'intérêt ou sur un procédé d'isolement des acides nucléiques de micro-organismes d'intérêt et des kits pour permettre l'isolement des micro-organismes d'intérêt et/ou des acides nucléiques de micro-organismes d'intérêt au sein d'un échantillon biologique liquide comprenant ou susceptible de comprendre, notamment, des micro-organismes d'intérêt, des cellules non ciblées et, éventuellement, des débris de micro-organismes d'intérêt et/ou de cellules non ciblées.

L'état de la technique est constitué par un certain nombre de publications scientifiques et de brevets. Ainsi en 1992, Baker et ses collaborateurs décrivent l'utilisation d'un réactif nommé saponine pour lyser de manière différentielle des cellules humaines et isoler le pathogène plasmodium du sang total. Ils ont démontré que la saponine avec une concentration finale faible, de l'ordre de 0,015 %, peut lyser des cellules humaines dans 20 µL de sang total tamponné au citrate et fournir assez de lysat propre pour faire une détection PCR.

La demande de brevet EP-A-0.745.849 a revisité cette approche différentielle d'utilisation de la saponine pour des volumes de sang total plus larges (5 mL) avec une solution finale concentrée en saponine de l'ordre de 0,020-0,125 %.

Une autre demande de brevet EP-A-2.185.681 propose une méthode de préparation des solutions de saponine, en utilisant une solution de saponine plus concentrée, c'est-à-dire avec une concentration finale comprise entre 2 et 10 % pour un volume de sang total de taille moyenne, soit 5 mL. Cette invention propose d'utiliser des tampons hypotoniques ou physiologiques pour de telles solutions de saponine.

Toutefois, de nos jours, aucune solution rapide n'est disponible pour isoler et identifier une très faible quantité de cellules pathogènes au sein de volumineux échantillons biologiques. Par exemple, pour la septicémie, le besoin est de pouvoir identifier de 1 à 10 unités formant des colonies (CFU, Colony-Forming Unit) de pathogènes dans 10 mL de sang total dans un délai de moins de 6 heures, soit dans les cas les plus difficiles 1 fg d'acide nucléique de pathogène ciblé pour plus de 800 µg d'acides nucléiques non ciblés (humain).

Les problèmes rencontrés dans le diagnostic des maladies infectieuses sont donc :
1) la complexité de l'échantillon matriciel incluant d'ordinaire très peu de cellules de pathogènes, d'une part, et un très grand nombre de cellules humaines, et autres cellules ou particules, d'autre part, qui doivent être éliminées pour ne pas s'immiscer dans l'isolement et l'identification des pathogènes, et
2) les limitations physiques pour isoler un très petit nombre de pathogènes à partir d'un très grand volume d'échantillon.
En résumé, le défi est d'éliminer le maximum d'éléments (cellules et/ou particules) non-ciblés en gardant le maximum de cibles pathogènes qui étaient initialement peu présentes afin d'accroître artificiellement leur concentration et faciliter leur détection.

L'invention décrite ici est une méthode qui peut isoler et identifier un petit nombre de pathogènes à partir de grands volumes d'échantillons biologiques (par exemple de l'ordre de 10ml) dans un délai relativement court. Ces pathogènes sont de manière non limitative des bactéries, des virus et des champignons. L'invention permet d'identifier d'aussi faibles quantités que 3 CFU dans 10 mL de sang total (soit 0,3 CFU/mL), avec une limite de détection jamais décrite dans la littérature. Sa nouveauté est basée sur :
1. Une limite de détection attractive jamais atteinte précédemment de l'ordre de 0,3 CFU / mL, en rapport au besoin clinique qui est de 0,1 à 1 CFU/mL.
2. Une capacité à utiliser différents sangs totaux tamponnés avec de l'EDTA ou du Citrate mais aussi avec de l'héparine. L'héparine est un réactif très utilisé à l'hôpital, mais souvent écarté dans les approches de biologie moléculaire du fait de sa capacité à empêcher l'amplification d'acides nucléiques et les réactions à base d'enzyme(s). Cette invention peut gérer le sang tamponné à l'héparine, ce qui n'est pas le cas en général des méthodes moléculaires décrites dans la littérature.
3. Une capacité à utiliser de grands volumes de sang total de 10 mL ou plus.
4. L'utilisation de polyéthylène glycol (PEG) pour améliorer la précipitation des pathogènes. La précipitation d'ADN ou de virus par le PEG est très bien décrite dans la littérature. Cependant, l'utilisation de PEG pour précipiter des cellules bactériennes ou de champignons tels que les levures est absente dans l'état de l'art. Etant donné que la saponine est un réactif semblable au détergent, les cellules ou particules pathogènes peuvent flotter à l'intérieur du tube ou avoir des difficultés à précipiter dans le fond d'un tube sous l'effet d'une centrifugation. Même si certains échantillons traités ne le nécessitent pas, l'ajout de PEG garantit une précipitation des cibles qui est constante sans risque de flottaison. De plus, cet ajout de PEG améliore le collage des culots contenant des pathogènes sur le tube plastique et la résistance aux lavages de surface.
5. Associer la lyse différentielle avec de la saponine avec des traitements à base de nucléases fortement concentrés ; en d'autres termes, lyser des cellules non-cibles qui vont libérer leurs acides nucléiques, qui dans un deuxième temps seront dégradés par un traitement aux nucléases. Cette lyse différentielle libère une grande quantité d'acides nucléiques humains. Typiquement 880 µg d'ADN humain peuvent être extraits de 10 ml de sang total. Ces acides nucléiques humains sont la source d'importants problèmes quand le but est de détecter un très petit nombre de copies d'acides nucléiques pathogènes :
   i) ils entrent en compétition lors de la purification des acides nucléiques cibles pathogènes en saturant les réactifs de capture d'acides nucléiques en raison de leur excès (comme par exemple les membranes de silice, les billes de silice magnétiques, les matrices de chromatographie ou d'affinité, etc.),
   ii) ils interférèrent pendant les amplifications des molécules pathogènes. En éliminant le maximum d'acides nucléiques humains, les limites de détection sont améliorées. Selon ces points critiques, l'invention utilise une grande quantité de nucléases pour éliminer un maximum d'acides nucléiques humains après la lyse par la saponine. La combinaison de saponine avec un traitement de nucléases fortement concentrées n'a jamais été décrite dans la littérature.
6. Enfin, nous avons démontré que la solution saponine tamponnée, avec un pH compris entre 6 et 9, permet d'éviter la précipitation d'éléments indésirables du sang, particulièrement des globules rouges. Les scientifiques ont précédemment utilisé des tampons près de pH 7 pour la solution saponine, mais sans jamais corréler l'impact de ce pH avec la stabilité de l'échantillon.

En résumé, la présente invention combine différentes utilisations de produits chimiques permettant d'atteindre une limite de détection de pathogène compatible avec les valeurs de détection d'une gamme clinique (0,1 à 1 CFU/mL). L'utilisation combinée de saponine avec du PEG et au moins une nucléase n'a jamais été décrite et permet une amélioration technique inconnue à ce jour. La combinaison de ces réactifs peut être complète (saponine + PEG + nucléase) ou partielle (saponine + nucléase ou saponine + PEG seulement ou encore PEG seul) pour préparer l'échantillon pour une analyse d'identification ou une détection.

A cet effet, la présente divulgation concerne un procédé d'isolement sélectif de micro-organismes d'intérêt au sein d'un échantillon biologique liquide comprenant ou susceptible de comprendre, notamment :
- des micro-organismes d'intérêt dont la membrane cellulaire ou la capside ne contient pas de cholestérol, et
- des éléments non ciblés, c'est-à-dire :
   - des cellules non ciblées dont la membrane cellulaire contient du cholestérol, et
   - éventuellement, des virus à enveloppes contenant du cholestérol, et
   - éventuellement des mycoplasmes contenant du cholestérol, et
   - éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol ou les enveloppes virales contenant du cholestérol ou les membranes de mycoplasme contenant du cholestérol,
b)réaliser un choc osmotique des cellules non-ciblées afin de les lyser spécifiquement,
c)ajouter une solution d'au moins une enzyme apte à lyser les acides nucléiques libres (ADN et/ou ARN) issus des éléments non ciblés lysés en solution dans l'échantillon, permettant d'obtenir sélectivement les micro-organismes d'intérêt.
La présente divulgation couvre également un procédé d'isolement sélectif de micro-organismes d'intérêt au sein d'un échantillon biologique liquide comprenant ou susceptible de comprendre, notamment :
- des micro-organismes d'intérêt dont la membrane cellulaire ou la capside ne contient pas de cholestérol, et
- des éléments non ciblés c'est-à-dire :
   - des cellules non ciblées dont la membrane cellulaire contient du cholestérol, et
   - éventuellement, des virus à enveloppes contenant du cholestérol, et
   - éventuellement des mycoplasmes contenant du cholestérol, et
   - éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol et/ou les enveloppes virales contenant du cholestérol et/ou les membranes des mycoplasmes contenant du cholestérol,
b) réaliser un choc osmotique des cellules non-ciblées afin de les lyser spécifiquement,
c) ajouter un agent de précipitation des micro-organismes d'intérêt non lysés en solution dans l'échantillon, permettant d'obtenir sélectivement les micro-organismes d'intérêt.
L'invention couvre un procédé d'isolement sélectif de micro-organismes d'intérêt au sein d'un échantillon biologique liquide comprenant ou susceptible de comprendre, notamment :
- des micro-organismes d'intérêt dont la membrane cellulaire ou la capside ne contient pas de cholestérol, et
- des éléments non ciblés c'est-à-dire :
   - des cellules non ciblées dont la membrane cellulaire contient du cholestérol, et
   - éventuellement, des virus à enveloppes contenant du cholestérol, et
   - éventuellement des mycoplasmes contenant du cholestérol, et
   - éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol et/ou les enveloppes virales contenant du cholestérol et/ou les membranes de mycoplasmes contenant du cholestérol,
b) réaliser un choc osmotique des cellules non-ciblées afin de les lyser spécifiquement,
c) ajouter un agent de précipitation des micro-organismes d'intérêt non lysés en solution dans l'échantillon, qui est du polyéthylène glycol et
d) ajouter une solution d'au moins une enzyme apte à lyser les acides nucléiques libres (ADN et/ou ARN) issus des éléments non ciblés lysés en solution dans l'échantillon, permettant d'obtenir sélectivement les micro-organismes d'intérêt.
L'étape c) de ce dernier procédé peut être réalisée indépendamment à l'étape a), après les étapes a) et b) ou après l'étape d) .

La présente divulgation concerne également un procédé d'isolement sélectif d'acides nucléiques d'intérêt au sein d'un échantillon biologique liquide comprenant ou susceptible de comprendre, notamment :
- des micro-organismes d'intérêt dont la membrane cellulaire ou la capside ne contient pas de cholestérol, et
- des éléments non ciblés, c'est-à-dire :
   - des cellules non ciblées dont la membrane cellulaire contient du cholestérol, et
   - éventuellement, des virus à enveloppes contenant du cholestérol, et
   - éventuellement des mycoplasmes contenant du cholestérol, et
   - éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol et/ou enveloppes virales contenant du cholestérol et/ou les membranes de mycoplasmes,
b) réaliser un choc osmotique des cellules non-ciblées afin de les lyser spécifiquement,
c) ajouter une solution d'au moins une enzyme apte à lyser les acides nucléiques libres (ADN et/ou ARN) issus des éléments non ciblés lysés en solution dans l'échantillon,
d) inactiver l'enzyme ajoutée à l'étape (c), et
e) rendre accessible les acides nucléiques de micro-organismes d'intérêt non dégradés par l'enzyme de l'étape (c).

Selon un cinquième mode de réalisation , la divulgation couvre un procédé d'isolement sélectif d'acides nucléiques d'intérêt au sein d'un échantillon biologique liquide comprenant ou susceptible de comprendre, notamment :
- des micro-organismes d'intérêt dont la membrane cellulaire ou la capside ne contient pas de cholestérol, et
- des éléments non ciblés c'est-à-dire :
   - des cellules non ciblées dont la membrane cellulaire contient du cholestérol, et
   - éventuellement, des virus à enveloppes contenant du cholestérol, et
   - éventuellement des mycoplasmes contenant du cholestérol, et
   - éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol et/ou les enveloppes virales contenant du cholestérol et/ou les membranes de mycoplasmes contenant du cholestérol,
b) réaliser un choc osmotique des cellules non-ciblées afin de les lyser spécifiquement,
c) ajouter un agent de précipitation des micro-organismes d'intérêt non lysés en solution dans l'échantillon, et
d) rendre accessible les acides nucléiques de micro-organismes d'intérêt non accessibles par l'action des étapes a) et b).

L'invention couvre également un procédé d'isolement sélectif d'acides nucléiques d'intérêt au sein d'un échantillon biologique liquide comprenant ou susceptible de comprendre, notamment :
- des micro-organismes d'intérêt dont la membrane cellulaire ou la capside ne contient pas de cholestérol, et
- des éléments non ciblés c'est-à-dire :
   - des cellules non ciblées dont la membrane cellulaire contient du cholestérol, et
   - éventuellement, des virus à enveloppes contenant du cholestérol, et
   - éventuellement des mycoplasmes contenant du cholestérol, et
   - éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol et/ou les enveloppes virales contenant du cholestérol et/ou les membranes de mycoplasmes contenant du cholestérol,
b) réaliser un choc osmotique des cellules non-ciblées afin de les lyser spécifiquement,
c) ajouter un agent de précipitation des micro-organismes d'intérêt non lysés en solution dans l'échantillon qui est du polyéthylène glycol, et
d) ajouter une solution d'au moins une enzyme apte à lyser les acides nucléiques libres (ADN et/ou ARN) issus des éléments non ciblés lysés en solution dans l'échantillon,
e) inactiver l'enzyme ajoutée à l'étape (d),
f) obtenir les acides nucléiques de micro-organismes d'intérêt :
   - non dégradés par l'enzyme de l'étape (d), et
   - non accessibles par l'action des étapes a) et b).
L'étape c) de ce dernier procédé peut être réalisée indépendamment à l'étape a), après les étapes a) et b) ou après l'étape d).

Selon un septième aspect, la présente divulgation concerne également un procédé de précipitation amélioré des micro-organismes d'intérêt choisis parmi les bactéries et les champignons (de préférence les levures), consistant à ajouter au moins un agent de précipitation, dans des échantillons biologiques liquides, le cas échéant préalablement traités par la saponine.

L'ajout d'agent(s) de précipitation permet d'améliorer le rendement de précipitation des micro-organismes contenus dans un échantillon biologique liquide.

Quel que soit le mode de réalisation, le procédé d'isolement est caractérisé en ce que la saponine est dans un volume au moins égal ou supérieur au volume de l'échantillon à concentration égale.

La concentration finale en saponine est supérieure à 0,02 % et inférieure ou égale à 20 %, de préférence comprise entre 0,05 % et 20%, plus préférentiellement entre 0,5 et 20% et encore plus préférentiellement entre 0,08 et 4%.

Le choc osmotique des cellules non ciblées afin de lyser spécifiquement ces dernières est une propriété inhérente à la mise en contact de l'échantillon biologique liquide avec la formulation de saponine telle que décrite à l'étape a). Cette formulation de saponine est utilisée en grand volume ce qui a pour effet :
- de fragiliser ou déstabiliser les membranes cellulaires contenant du cholestérol, à savoir les membranes des cellules non-ciblées, et en même temps
- d'induire une pression osmotique qui va mener à la turgescence des cellules non-ciblées et à leur lyse.
En d'autres termes, les étapes a) et b) peuvent se réécrire en une seule étape consistant à mettre en contact l'échantillon biologique liquide avec une formulation de saponine afin de lyser spécifiquement les membranes cellulaires des cellules non-ciblées contenant du cholestérol et les enveloppes virales contenant du cholestérol et les membranes des mycoplasmes contenant du cholestérol.

Le procédé d'isolement est aussi caractérisé en ce que la saponine est constituée par un triterpénoïde.
La saponine est spécifique des membranes ou enveloppes contenant du cholestérol.

Dans le cadre du procédé d'isolement de micro-organismes d'intérêt et du procédé de précipitation amélioré de micro-organismes d'intérêt selon la divulgation, l'agent de précipitation utilisé est choisi parmi le PEG, le glycogène et les acides nucléiques (ADN/ARNt..). Il est également possible de les utiliser en mélange.

L'agent de précipitation utilisé dans le cadre de l'invention est le PEG.

Dans le cadre du procédé d'isolement d'acides nucléiques de micro-organismes d'intérêt, l'agent de précipitation peut être constitué par le polyéthylène glycol (PEG).

Les concentrations d'agent de précipitation utilisées dans le cadre de l'invention sont comprises entre 0,1% et 20%, de préférence entre 1% et 20%.

A l'issue du procédé d'isolement des micro-organismes d'intérêt, quelle que soit sa mise en œuvre, il est possible de traiter le milieu obtenu pour isoler davantage les micro-organismes d'intérêt, permettant ainsi leur détection. Ceci se fait grâce à des techniques cellulaires classiques, par exemple des techniques de cytologie (cytométrie de flux ou autres), des techniques d'immunologie (technologie avec les anticorps ou les phages pour une détection par immunodosage) ou des techniques de cultures cellulaires classiques.
Ainsi, il est possible d'appliquer le milieu obtenu pour une mise en culture classique sur un milieu approprié, de préférence sur un milieu solide (par exemple un milieu gélosé nutritif) en boîte de Pétri pendant au moins 24h, par exemple 48h pour les levures, à une température adaptée, par exemple 30°C pour les levures, de préférence 37°C, ou tout milieu approprié à la croissance des micro-organismes d'intérêt. Toute technique et protocole connus permettant la croissance des micro-organismes puis leur individualisation/isolement peuvent également être utilisés.

Selon certaines variantes de réalisation du procédé d'isolement, l'enzyme apte à lyser les acides nucléiques libres est une enzyme qui est ensuite inactivée :
- chimiquement par l'ajout d'EDTA et/ou d'EGTA et/ou de DTT et/ou de β-mercaptoéthanol et/ou de DEPC, et/ou de la guanidine, et/ou
- physiquement par l'augmentation de la température entre 40 et 100°C en présence ou non de détergents, tels que le Sodium Docécyl Sulfate (SDS).

Dans les cas où le procédé d'isolement selon l'invention ne comprend qu'une seule enzyme apte à lyser les acides nucléiques libres (ADN et/ou ARN) issus des éléments non ciblés lysés en solution dans l'échantillon, cette enzyme est une ADNase.

Selon certaines variantes de réalisation du procédé d'isolement où est ajoutée au moins une enzyme apte à lyser les acides nucléiques libres, c'est-à-dire lors de l'étape (c) lorsque le procédé utilisé ne comprend pas d'étape d'ajout d'agent de précipitation ou lors de l'étape (d) lorsque le procédé comprend une étape d'ajout d'agent de précipitation, l'enzyme apte à lyser les acides nucléiques libres est une enzyme qui peut être inactivée :
- chimiquement par l'ajout d'EDTA et/ou d'EGTA et/ou de DTT et/ou de β-mercaptoéthanol et/ou de DEPC, et/ou de la guanidine, et/ou
- physiquement par l'augmentation de la température entre 40 et 100°C en présence ou non de détergents, tels que le Sodium Docécyl Sulfate (SDS).

Dans les procédés d'isolement de micro-organismes d'intérêts et d'isolement d'acides nucléiques de micro-organismes d'intérêt comprenant une étape d'ajout d'agent de précipitation et une étape d'ajout d'au moins une enzyme apte à lyser les acides nucléiques libres (ADN et/ou ARN) issus des éléments non ciblés lysés en solution dans l'échantillon, ces deux étapes n'ont pas un ordre défini et peuvent être interverties dans le déroulement du procédé.
Dans le cadre du procédé d'isolement d'acides nucléiques de micro-organismes d'intérêt comprenant une étape d'inactivation de l'enzyme apte à lyser les acides nucléiques libres, l'agent de précipitation peut être ajouté après cette étape d'inactivation.

L'invention concerne également un procédé pour l'isolement de micro-organismes d'intérêt ou pour l'isolement d'acides nucléiques de micro-organismes d'intérêt ou pour la précipitation de micro-organismes d'intérêt, dans un échantillon biologique liquide, de préférence de sang, caractérisé en ce que lors du procédé, le pH est maintenu dans une fourchette entre 5 et 10, préférentiellement entre 6 et 9, par l'ajout d'une solution :
- basique si la valeur du pH est inférieure à 5, préférentiellement inférieure à 6,
- acide si la valeur du pH est supérieure à 10, préférentiellement supérieure à 9,
afin que le pH soit compris dans la fourchette.

Selon une variante d'utilisation, la divulgation utilise une formulation de saponine conduisant à une concentration finale supérieure à 0,02% et inférieure ou égale à 20%, préférentiellement supérieure à 0,05% et inférieure ou égale à 20% et/ou un agent précipitant (ou agent de précipitation) à une concentration de 0,1 à 20%, de préférence 0,1 à 4%, plus préférentiellement encore de 0,5 à 4% , et/ou une enzyme apte à lyser les acides nucléiques libres (ADN et/ou ARN) contenant entre 500 et 20000 unités enzymatiques.

Selon un autre de ses aspects, l'invention couvre l'utilisation d'une formulation de saponine et d'une solution d'au moins une enzyme apte à lyser les acides nucléiques libres (ADN et/ou ARN) et d'au moins un agent de précipitation qui est du PEG, pour l'isolement de micro-organismes d'intérêt ou d'acides nucléiques de micro-organismes d'intérêt, dans un échantillon biologique liquide.

Selon un autre de ses aspects, la présente invention couvre l'utilisation du PEG pour la précipitation de micro-organismes d'intérêt, de préférence des bactéries et des champignons (de préférence les levures), dans des échantillons biologiques liquides, le cas échéant préalablement traités par la saponine.

L'invention concerne encore un test de diagnostic basé sur un procédé d'isolement de micro-organismes d'intérêt, tel que décrit ci-dessus, ou sur les utilisations pour l'isolement des micro-organismes d'intérêt, telles que décrites ci-dessus, ou sur un procédé de précipitation de micro-organismes tel que décrit ci-dessus.

L'invention concerne également un test de diagnostic basé sur un procédé d'isolement d'acides nucléiques de micro-organismes d'intérêt, tel que décrit ci-dessus, ou sur les utilisations pour l'isolement des acides nucléiques de micro-organismes d'intérêt, telles que décrites ci-dessus.

Le diagnostic peut être réalisé par des techniques classiques connues de l'homme du métier mettant, par exemple, en œuvre des techniques classiques de détection utilisées en microbiologie, des techniques d'immunodosage ou des techniques de biologie moléculaire classiques comme la PCR, la NASBA...

L'invention concerne également un kit de diagnostic pour permettre l'isolement de micro-organismes d'intérêt au sein d'un échantillon biologique liquide comprenant ou susceptible de comprendre, notamment, des micro-organismes d'intérêt, des cellules non ciblées et, éventuellement, des virus à enveloppes, des mycoplasmes et/ou des débris de micro-organismes d'intérêt et/ou de cellules non ciblées, le kit comprenant :
(a)un récipient, et
(b) au moins une formulation de saponine, et
(c)au moins une solution d'un agent de précipitation, qui est le polyéthylène glycol (PEG)
(d) au moins une enzyme apte à lyser les acides nucléiques, et
(e) le nécessaire pour détecter les micro-organismes d'intérêt et/ou les acides nucléiques de micro-organismes d'intérêt.

L'invention concerne enfin un kit de diagnostic pour permettre l'isolement des acides nucléiques de micro-organismes d'intérêt au sein d'un échantillon biologique liquide comprenant ou susceptible de comprendre, notamment, des micro-organismes d'intérêt, des cellules non ciblées et, éventuellement, des virus à enveloppes, des mycoplasmes et/ou des débris de micro-organismes d'intérêt et/ou de cellules non ciblées, le kit comprenant:
(a)un récipient, et
(b) au moins une formulation de saponine, et
(c) au moins une solution d'un agent de précipitation, qui est le polyéthylène glycol (PEG),
(d) au moins une enzyme apte à lyser les acides nucléiques, et
(e) le nécessaire pour détecter les micro-organismes d'intérêt et/ou les acides nucléiques de micro-organismes d'intérêt.

Les deux kits décrits ci-dessus peuvent comprendre en outre (d) au moins une solution d'au moins une enzyme apte à lyser les acides nucléiques.

Le kit comprend en outre :
(c) ou (d') au moins une solution acide et/ou au moins une solution basique, et/ou
(d) de l'EDTA et/ou de l'EGTA et/ou du DTT et/ou du β-mercaptoéthanol et/ou du DEPC, et/ou de la guanidine et/ou
(e) au moins un détergent ou un agent anionique.

Dans la présente description, la ou les enzymes permettant de lyser les acides nucléiques sont des enzymes connues et classiquement utilisées par l'homme du métier, telles que la DNAse I, la RNAseIf... et l'étape consistant à rendre accessible les acides nucléiques des micro-organismes d'intérêt est effectuée de façon classique et connue de l'homme du métier, par exemple par une lyse mécanique puis une purification des acides nucléiques et une amplification PCR ou toute autre technique connue.

Dans la suite de cette demande de brevet, les termes utilisés reçoivent les définitions suivantes :
- Par « enveloppe virale », on entend l'enveloppe des virus enveloppés qui, elle, contient du cholestérol contrairement à la capside des virus à capside qui n'en contient pas. L'enveloppe virale est donc sensible à la saponine.
- Par « déstabiliser les membranes cellulaires et/ou enveloppes virales et/ou les membranes des mycoplasmes », on entend les mécanismes physicochimiques conduisant à une perte de régulation de l'osmolarité au niveau membranaire et/ou de l'enveloppe virale et/ou de la membrane d'un mycoplasme, une perturbation du transport membranaire et l'apparition potentielle de pores au niveau de la membrane cellulaire, mycoplasmique ou de l'enveloppe virale.
- Les termes « micro-organismes d'intérêt » comprennent tous micro-organismes qui ne contiennent pas de cholestérol accessible, lesquels sont potentiellement pathogènes, notamment pour l'homme. Ces micro-organismes regroupent des virus (à l'exception des virus à enveloppe), des bactéries, des champignons (levures) mais aussi des animaux microscopiques.
- Les « acides nucléiques d'intérêt » correspondent aux acides nucléiques (ADN et ARN) contenus dans les cellules ou particules des « micro-organimes d'intérêt » définis ci-dessus.
- Par « cellules non ciblées », il faut comprendre toutes les cellules d'organismes vivants qui ne contiennent pas d'« acides nucléiques d'intérêts » comme définis ci-dessus.
- L'abréviation « EDTA » correspond à l'acide éthylènediamine tétraacétique (correspondant en anglais *à EthyleneDiamineTetraacetic Acid*).
- L'abréviation « EGTA » correspond à l'acide tétraacétique éthylène glycol (soit en anglais *Ethylene Glycol Tetraacetic Acid*).
- L'abréviation « DTT » correspond au Dithiothreitol.
- L'abreviation « DEPC » correspond à Diéthylpyrocarbonate.
- L'abréviation CFU est pour Colony-Forming Unit ou unité formant des colonies
- Par le terme « détergents » on entend toutes classes de molécules pouvant induire une modification physicochimiques d'autres molécules. Ces détergents peuvent être de nature chimique comme le SDS, le tween-20, le tritonx-100, le brij97 ou enzymatiques.
- Par « échantillon biologique liquide », il faut comprendre un échantillon liquide susceptible de contenir les « micro-organismes d'intérêt » sélectionné parmi le groupe suivant : liquide amniotique, humeur aqueuse, bile, sang, sécrétion mamellaire, lavage broncho alvéolaire, liquide cérébrospinal, chyle, chyme, fèces, liquide interstitiel, lymphe, menstruations, mucus, plasma, liquide pleural, pus, salive, sébum, sperme, sérum, crachat, sueur, fluide synovial, larme, urine et humeur vitrée.
Par ailleurs, il doit être clair que le procédé d'isolement sélectif d'acides nucléiques d'intérêt tel que décrit ci-dessus ainsi que son utilisation permettent l'isolement des micro-organismes d'intérêt, dans la mesure où les acides nucléiques d'intérêt correspondent aux acides nucléiques (ADN et/ou ARN) contenus dans les cellules ou particules des micro-organismes d'intérêt. En d'autres termes, l'isolement sélectif des acides nucléiques d'intérêt permet l'isolement des micro-organismes d'intérêt dont les acides nucléiques d'intérêt proviennent.

Les exemples ci-joints sont présentés pour démontrer l'efficacité du procédé selon l'invention et sont donnés à titre illustratif et non à titre limitatif.

### Exemple 1 : Détection de 5 et 10 CFU de Pseudomonas aeruginosa dans 10 mL de sang total traités avec une solution de saponine 4%

À l'intérieur d'un tube plastique de 50 mL, 20 mL de sang total traité par l'EDTA ont été inoculés avec 20, 10, 2 et 0 (contrôle négatif) CFU de *Pseudomonas aeruginosa.* Le nombre de CFU insérées dans le sang a été vérifié par étalement sur milieux gélosés en boîte de Petri. Les 20 mL de sang inoculés ont été divisés en deux et chaque volume de 10 mL a été déposé dans deux tubes plastiques de 50 mL pour avoir des duplicatas. Quarante millilitres d'une solution filtrée de 4 % de saponine, 50 mM Tris-HCl à pH 8,0 et 4% de PEG-8000 ont été ajoutés au sang inoculé. Les tubes ont été agités par inversion deux fois, incubés à température ambiante pendant 5 minutes et centrifugés à 12000 g pendant 10 minutes. Le surnageant a été éliminé et le culot collant a été lavé trois fois avec 15 mL de 4 % PEG-8000 qui évite le décrochage du culot. Un volume de 200 µL de Tris-HCl à 10 mM à un pH de 7,5 a été ajouté au culot.

Par la suite, 10 µL de DNAse I (500 u/µl ; Roche) et 2 µL RNAse If (50 u/µL ; New England Biolabs) ont été mis dans les tubes. Les culots ont été digérés par les enzymes pendant 10 minutes avec deux agitations par vortex après 2 minutes et 4 minutes d'incubation. Après la digestion par les nucléases, 10 µL d'EDTA à 0,5 M et pH 8,0 ont été ajoutés aux tubes. Les échantillons ont été transférés dans des microtubes d'une contenance de 1,5 mL, contenant 200 mg de billes de verre de diamètre de 1 mm et 50 mg de billes de zirconium de 0,1 mm de diamètre. Les microtubes ont été chauffés 10 minutes à 80°C. On a ensuite ajouté 20 µL de Protéinase K (Novagen) et 40 µL à 10 % de SDS aux tubes. Les microtubes ont été incubés 5 minutes à température ambiante et chauffés 5 minutes à 80°C.

La lyse mécanique des cellules de *Pseudomonas* a été réalisée en agitant les tubes contenant les billes pendant 20 minutes à l'aide d'un vortex. L'ADN présent dans le supernageant de lyse a été purifié à l'aide du kit Nucleospin Blood^{®} de Macherey-Nagel. Une amplification PCR quantitative a été réalisée avec l'utilisation de l'éluat au complet, soit 40 µl. Les échantillons à 5 CFU de *Pseudomonas aeruginosa* ont bien été détectés sur un réplica à l'aide de l'invention, et une détection partielle d'un réplica sur deux a été observée pour 1 CFU insérée, comme cela est bien présenté sur le Tableau I suivant :

**Tableau I : Evaluation de la limite de détection pour Pseudomonas aeruginosa à l'aide du procédé selon l'invention**

| **Echantillon** | **Cq** | **Nombre de réplica détecté** |
|---|---|---|
| 10 CFU total | 38,78 | 2/2 |
| 10 CFU total | 37, 74 | |
| 5 CFU total | 42,18 | 2/2 |
| 5 CFU total | 37,84 | |
| 1 CFU total | 41,73 | 1/2 |
| 1 CFU total | N/A* | |
| Contrôle négatif | N/A* | 0/2 |
| Contrôle négatif | N/A* | |

| | | |
|---|---|---|
| * : aucun signal de fluorescence n'a été détecté après 50 cycles d'amplification. | | |

### Exemple 2 : Détection de 28 et 140 CFU de Candida albicans dans 10 mL de sang total traités avec une solution de saponine 4%

À l'intérieur d'un tube plastique de 50 mL, 20 mL de sang total traité par l'EDTA ont été inoculés avec 140, 28 et 0 (contrôle négatif) CFU de *Candida albicans.* Le nombre de CFU insérées dans le sang a été vérifié par étalement sur milieux gélosés en boîte de Petri. Les 20 mL de sang inoculés ont été divisés en deux et chaque volume de 10 mL a été déposé dans deux tubes plastiques de 50 mL pour avoir des duplicatas. Quarante millilitres d'une solution filtrée de 4 % de saponine, 50 mM Tris-HCl à pH 8,0 et 4% de PEG-8000 ont été ajoutés au sang inoculé. Les tubes ont été agités par inversion deux fois, incubés à température ambiante pendant 5 minutes et centrifugés à 12000 g pendant 10 minutes. Le surnageant a été éliminé et le culot a été lavé trois fois avec 15 mL de 4 % PEG-8000 qui évite le décrochage du culot. Un volume de 200 µL de Tris-HCl à 10 mM à un pH de 7,5 a été ajouté au culot. Par la suite, 10 µL de DNAse I (500 µ/µL ; Roche) et 2 µL RNAse If (50 µ/µL ; New England Biolabs) ont été mis dans les tubes. Les culots ont été digérés par les enzymes pendant 10 minutes avec deux agitations par vortex après 2 minutes et 4 minutes d'incubation. Après la digestion par les nucléases, 10 µL d'EDTA à 0,5 M et pH 8,0, un volume de 40 µl de SDS 10% et 20 µl de Protéinase K ont été ajoutés aux tubes. Les échantillons ont été transférés dans des microtubes d'une contenance de 1,5 mL, contenant 200 mg de billes de verre de diamètre de 1 mm et 50 mg de billes de zirconium de 0,1 mm de diamètre. Les microtubes ont été chauffés 60 minutes à 80°C. La lyse mécanique des cellules de *Candida albicans* a été réalisée en agitant les billes pendant 20 minutes à l'aide d'un vortex. L'ADN présent dans le surnageant de lyse a été purifié à l'aide du kit Nucleospin Blood^{®} de Macherey-Nagel et purifié une seconde fois à l'aide du kit gDNA Clean-up XS^{®} de Macherey-Nagel. Une amplification PCR quantitative a été réalisée avec l'utilisation de l'éluat au complet soit 40 µL. Les échantillons à 28 CFU de *Candida albicans* ont été détectés sur un réplica à l'aide de l'invention, comme cela est bien représenté sur le Tableau 2 suivant :

**Tableau 2 : Détection pour Candida albicans à l'aide du procédé selon l'invention**

| **Echantillon** | **Cq** | **Nombre de réplica détecté** |
|---|---|---|
| 140 CFU total | 37, 44 | 2/2 |
| 140 CFU total | 36,78 | |
| 28 CFU total | 40,08 | 2/2 |
| 28 CFU total | 38,42 | |
| Contrôle négatif | N/A* | 0/1 |

| | | |
|---|---|---|
| * : aucun signal de fluorescence n'a été détecté après 50 cycles d'amplification. | | |

### Exemple 3 : Détection de 20000 virions d'Adénovirus 5 humain dans 10 mL de sang total traités avec une solution de 4% de saponine

À l'intérieur d'un tube plastique de 50 mL, 10 mL de sang total traité à l'EDTA ont été inoculés avec 20000 virons d'Adénovirus 5 humain. Quarante millilitres d'une solution filtrée de 4 % de saponine, avec 50 mM Tris-HCl à pH 8,0 et 4% de PEG-8000 ont été ajoutés au sang inoculé. Les tubes ont été agités par inversion deux fois, incubés à température ambiante pendant 5 minutes et centrifugés à 12000 g pendant 10 minutes. Le surnageant a été éliminé et le culot été lavé trois fois avec 15 mL de 4 % PEG-8000 qui évite le décrochage du culot. Un volume de 200 µL de Tris-HCl à 10 mM et à un pH de 7,5 a été ajouté au culot. Par la suite, 10 µL de DNAse I (500 µ/µl ; Roche) et 2 µL RNAse If (50 µ/µL ; New England Biolabs) ont été mis dans les tubes. Les culots ont été digérés par les enzymes pendant 10 minutes avec deux agitations par vortex après 2 minutes et 4 minutes d'incubation. Après la digestion par les nucléases, 10 µL d'EDTA 0,5 M, pH 8,0 et 40 µl de SDS 10% ont été ajoutés aux tubes. Les échantillons ont été transférés dans des microtubes d'une contenance de 1,5 mL, contenant 200 mg de billes de verre de diamètre de 1 mm et 50 mg de billes de zirconium de 0,1 mm de diamètre. Les microtubes ont été chauffés 10 minutes à 80°C. La lyse mécanique des virions a été réalisée en agitant les tubes contenant les billes pendant 20 minutes à l'aide d'un vortex. L'ADN présent dans le surnageant de lyse a été purifié à l'aide du kit Nucleospin Blood^{®} de Macherey-Nagel. Les acides nucléiques ont été élués avec 40µl. Une amplification PCR quantitative a été réalisée avec l'utilisation de 10 µl d'éluat. Les 20000 virions ont bien été détectés à l'aide de l'invention, voir Tableau 3 suivant :

**Tableau 3 : Evaluation de la limite de détection d'Adénovirus 5 humain à l'aide de l'invention**

| **Echantillon** | **Cq** | **Nombre de réplica détecté** |
|---|---|---|
| 20000 virions total | 34,7 | 1/1 |

### Exemple 4 : Efficacité de lyse de cellules humaines sanguines par différentes concentrations de saponine.

À l'intérieur d'un tube plastique de 50 mL, 10 mL de sang total traité par l'EDTA ont été inoculés avec 24 et 0 (contrôle négatif) CFU de *Pseudomonas aeruginosa.* Le nombre de CFU insérées dans le sang a été vérifié par étalement sur milieux gélosés en boîte de Petri. Quarante millilitres d'une solution filtrée de saponine, 50 mM Tris-HCl à pH 8,0 et 4% de PEG-8000 ont été ajoutés au sang inoculé. Les concentrations finales de saponine étaient de 0,005%, 0,02%, 0.08% et 0,4%. Chaque concentration a été testée en duplicata. Les tubes ont été agités par inversion trois fois, incubés à température ambiante pendant 10 minutes et centrifugés à 12000 g pendant 10 minutes. Le surnageant a été éliminé et le culot collant a été lavé trois fois avec 15 mL de 4 % PEG-8000 qui évite le décrochage du culot. Un volume de 400 µL de Tris-HCl à 10 mM à un pH de 7,5, MgCl2 2,5mM, CaCl2 0,5 mM, DNAse I (Roche) 5000u, RNAse If (New England Biolabs) 100u a été ajouté au culot. Les culots ont été incubés à 32°C sous agitation pendant 90 minutes. Les échantillons ont été transférés dans des microtubes contenant 10 µL d'EDTA à 0,5 M pH 8,0 et 400 µl de tampon B3 (Macherey-Nagel). Les microtubes ont été chauffés 10 minutes à 80°C puis refroidis 5 min dans la glace. 5 µg de lysine ont été ajoutés aux tubes. Les microtubes ont été incubés 10 minutes supplémentaires dans la glace puis ont été traités par le kit Nucleospin Blood^{®} de Macherey-Nagel selon les conditions du fournisseur en doublant toutefois les quantités de proteinase K et d'éthanol pour respecter les ratios de réactifs. Une amplification PCR quantitative a été réalisée avec l'utilisation de 2µl d'éluat pour la détection des ADN humains et 38 µL pour les PCR détectant l'ADN de *Pseudomonas aeruginosa.*

La majorité des tubes ont été positifs pour *Pseudomonas aeruginosa* (les contrôles négatifs, sangs non-inoculés, traités à 0.4% de saponine étant bien négatifs). L'exemple montre qu'il est possible de détecter *Pseudomonas aeruginosa* pour des concentrations de saponine bien inférieures à 0.4%.

Les amplifications pour les concentrations finales de saponine égales ou supérieures à 0.08% n'ont pas réussi à détecter d'ADN humain ou des quantités extrêmement faibles (Tableau 4). Au contraire, pour les concentrations finales de saponine égales ou inférieures à 0.02%, les quantités d'ADN humains détectés sont très élevées (Cq autour de 28). Ceci démontre qu'à une concentration finale de saponine inférieure ou égale à une valeur de 0.02%, les globules blancs du sang ne sont plus correctement lysés alors qu'ils sont très bien lysés avec une concentration finale de 0,08% ou 0,4% de saponine.

**Tableau 4 : Evaluation de la lyse des cellules humaines sanguines**

| **Echantillon** | **Cq (ADN humain)** | **Nombres de tubes positifs pour *P*. *aeruginosa*** |
|---|---|---|
| Contrôle négatif 0.4% saponine | Non détecté* | 0/2 (Contrôles) |
| Contrôle négatif 0.4% saponine | Non détecté* | |
| 0.4% saponine | 39, 16 | 2/2 |
| 0.4% saponine | Non détecté* | |
| 0.08% saponine | Non détecté* | 1/2 |
| 0.08% saponine | Non détecté* | |
| 0.02% saponine | 27,35 | 2/2 |
| 0.02% saponine | 27,99 | |
| 0.005% saponine | 28,63 | 1/2 |
| 0.005% saponine | 29,27 | |

| | | |
|---|---|---|
| * : aucun signal de fluorescence n'a été détecté après 50 cycles d'amplification. | | |

### Exemple 5 : Détection de 21 CFU de Pseudomonas aeruginosa dans 10 mL de sang total traités avec une solution de 0.4% de saponine.

À l'intérieur d'un tube plastique de 50 mL, 10 mL de sang total traité par l'EDTA ont été inoculés avec 21 (5 tubes) et 0 (contrôle négatif, 1 tube) CFU de *Pseudomonas aeruginosa.* Le nombre de CFU insérées dans le sang a été vérifié par étalement sur milieux gélosés en boîte de Petri. Quarante millilitres d'une solution filtrée de 0.4% saponine, 50 mM Tris-HCl à pH 8,0 et 4% de PEG-8000 ont été ajoutés au sang inoculé. Les tubes ont été agités par inversion trois fois, incubés à température ambiante pendant 10 minutes et centrifugés à 12000 g pendant 10 minutes. Le surnageant a été éliminé et le culot collant a été lavé trois fois avec 15 mL de 4 % PEG-8000 qui évite le décrochage du culot. Un volume de 800 µL de Tris-HCl à 10 mM à un pH de 7,5, MgCl2 2,5mM, CaCl2 0,5 mM, DNAse I (Roche) 5000u, RNAse If (New England Biolabs) 100u a été ajouté au culot. Les culots ont été incubés à 32°C sous agitation pendant 90 minutes. 10 µL d'EDTA à 0,5 M pH 8,0 et 400 µl de tampon B3 (Macherey-Nagel) ont étaient ajoutés puis les tubes ont été chauffés 10 minutes à 80°C puis refroidis 5 min dans la glace. 5 µg de lysine ont été ajoutés aux tubes. Les tubes ont été incubés 10 minutes supplémentaires dans la glace puis ont été traités par le kit Nucleospin Blood^{®} de Macherey-Nagel selon les conditions du fournisseur en quadruplant toutefois les quantités de proteinase K et d'éthanol pour respecter les ratios de réactifs. Une amplification PCR quantitative a été réalisée avec l'utilisation de 2µl d'éluat pour la détection des ADN humains et 38 µL pour les PCR détectant l'ADN de *Pseudomonas aeruginosa.*

**Tableau 5 : PCR quantitative de detection de l'ADN de P. aeruginosa**

| **Echantillon** | **Cq (ADN *P. aeru.*)** | **Nombres de tubes positifs pour *P*. *aeruginosa*** |
|---|---|---|
| Contrôle négatif | Non détecté* | 0/1 |
| 21 CFU total | 36,60 | 5/5 |
| 21 CFU total | 36,17 | |
| 21 CFU total | 37,21 | |
| 21 CFU total | 38,81 | |
| 21 CFU total | 38,36 | |

| | | |
|---|---|---|
| * : aucun signal de fluorescence n'a été détecté après 50 cycles d'amplification. | | |

### Exemple 6 : Détection par croissance sur milieu solide de 117 CFU de Candida albicans à partir de 10 mL de sang total traités avec 4% de saponine

À l'intérieur d'un tube plastique de 50 mL, 10 mL de sang total traité par l'EDTA ont été inoculés avec 117 CFU de *Candida albicans.* Le nombre de CFU inséré dans le sang a été vérifié par étalement sur milieux gélosés en boîte de Petri. Quarante millilitres d'une solution filtrée de 4 % de saponine, 50 mM Tris-HCl à pH 8,0 et 4% de PEG-8000 ont été ajoutés au sang inoculé. Les tubes ont été agités par inversion deux fois, incubés à température ambiante pendant 5 minutes et centrifugés à 12000 g pendant 10 minutes. Les culots obtenus ont été resuspendus avec 212µl de Tryptone sel (AES) ou 212 µl de mix de nucléases (Tris 10 mM pH7.5 ; DNAse I (Roche) 5000 u ; RNAse If (New England Biolabs) 100u), incubés 10 minutes à température pièce puis étalés sur un milieu solide en boite de petri SDC (bioMérieux). Le nombre de colonies a ensuite été compté après une incubation à 30°C pendant 48 heures. Le Tableau 6 montre qu'en moyenne 66% de CFU ont été retrouvés après traitement à la saponine avec ou sans traitement aux nucléases.

**Tableau 6 : Enumération sur boite de colonies de Candida albicans après traitement à 4% de saponine**

| **Echantillon** | **Resuspension** | **Nombre de colonies** | **Moyenne** |
|---|---|---|---|
| 117 CFU total | Tryptone-sel | 84 | 78.5 (67% de 117) |
| 117 CFU total | Tryptone-sel | 73 | |
| 117 CFU total | Mix nucléases | 87 | 76 (65% de 117) |
| 117 CFU total | Mix nucléases | 65 | |

| | | | |
|---|---|---|---|
| * : aucun signal de fluorescence n'a été détecté après 50 cycles d'amplification. | | | |

### Exemple 7 : Effet facilitateur du PEG sur la précipitation par centrifugation de Pseudomonas aeruginosa présent dans du sang

200µl de sang total traité par l'EDTA ont été répartis à l'intérieur de tube plastique de 1.5 mL. 1mL de MgCl2 10mM ou 1mL de MgCl2 supplémenté à 4% de PEG final ont été ajoutés aux tubes. Par la suite, ces tubes ont été inoculés avec 129 CFU de *Pseudomonas aeruginosa.* Les tubes ont été vortexés 5 secondes puis centrifugés 10 minutes à 5000g. Les culots ont, par la suite, été resuspendus avec 100µl de Tryptone sel puis étalés sur milieu solide en boite de Petri TSA (bioMérieux). Les boites ont été incubées 24h à 37°C avant énumération. L'ajout de PEG a contribué à une amélioration de 23.5% du rendement de précipitation (Tableau 7).

**Tableau 7 : Enumération sur boite de colonies de Pseudomonas aeruginosa après centrifugation avec ou sans présence de PEG**

| **Echantillon** | **Solution de précipitation** | **Nombre de colonies** | **Moyenne** |
|---|---|---|---|
| 129 CFU total | MgCl2 | 66 | 70 (54% de 129) |
| 129 CFU total | MgCl2 | 74 | |
| 129 CFU total | MgCl2+PEG | 100 | 100 (77.5% de 129) |
| 129 CFU total | MgCl2+PEG | 100 | |

| | | | |
|---|---|---|---|
| * : aucun signal de fluorescence n'a été détecté après 50 cycles d'amplification. | | | |

## Revendications

1. Procédé d'isolement sélectif de micro-organismes d'intérêt au sein d'un échantillon biologique liquide comprenant ou susceptible de comprendre, notamment :
• des micro-organismes d'intérêt dont la membrane cellulaire ou la capside ne contient pas de cholestérol, et
• des éléments non ciblés, c'est-à-dire :
- des cellules non ciblées dont la membrane cellulaire contient du cholestérol, et
- éventuellement, des virus à enveloppes contenant du cholestérol, et
- éventuellement des mycoplasmes contenant du cholestérol, et
- éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol ou les enveloppes virales contenant du cholestérol ou les membranes de mycoplasmes contenant du cholestérol,
b) réaliser un choc osmotique des cellules non-ciblées afin de les lyser spécifiquement,
c) ajouter un agent de précipitation des micro-organismes d'intérêt non lysés en solution dans l'échantillon, qui est du polyéthylène glycol,
d) ajouter une solution d'au moins une enzyme apte à lyser les acides nucléiques libres (ADN et/ou ARN) issus des éléments non ciblés lysés en solution dans l'échantillon, permettant d'obtenir sélectivement les micro-organismes d'intérêt.

2. Procédé d'isolement sélectif, selon la revendication **1,** dans lequel l'étape consistant à ajouter un agent de précipitation des micro-organismes d'intérêt non lysés en solution dans l'échantillon est réalisée à l'étape a), après les étapes a) et b) ou après l'étape d).

3. Procédé d'isolement sélectif d'acides nucléiques d'intérêt au sein d'un échantillon biologique liquide comprenant ou susceptible de comprendre, notamment :
• des micro-organismes d'intérêt dont la membrane cellulaire ou la capside ne contient pas de cholestérol, et
• des éléments non ciblés, c'est-à-dire :
- des cellules non ciblées dont la membrane cellulaire contient du cholestérol, et
- éventuellement, des virus à enveloppes contenant du cholestérol, et
- éventuellement des mycoplasmes contenant du cholestérol, et
- éventuellement, des débris de micro-organismes d'intérêt et/ou de cellules non ciblées,
le procédé comprenant les étapes suivantes :
a) mettre en contact l'échantillon biologique liquide avec une formulation de saponine, afin de déstabiliser les membranes cellulaires contenant du cholestérol et/ou les enveloppes virales contenant du cholestérol et/ou les membranes de mycoplasmes contenant du cholestérol,
b) réaliser un choc osmotique des cellules non-ciblées afin de les lyser spécifiquement,
c) ajouter un agent de précipitation des micro-organismes d'intérêt non lysés en solution dans l'échantillon, qui est du polyéthylène glycol,
d) ajouter une solution d'au moins une enzyme apte à lyser les acides nucléiques libres (ADN et/ou ARN) issus des éléments non ciblés lysés en solution dans l'échantillon,
e) inactiver l'enzyme ajoutée à l'étape (d), et
f) obtenir les acides nucléiques de micro-organismes d'intérêt :
• non dégradés par l'enzyme de l'étape (d), et
• non accessibles par l'action des étapes a) et b).

4. Procédé d'isolement sélectif d'acides nucléiques d'intérêt au sein d'un échantillon biologique liquide, selon la revendication 3, dans lequel l'étape consistant à ajouter un agent de précipitation des micro-organismes d'intérêt non lysés en solution dans l'échantillon, est réalisée à l'étape a), après les étapes a) et b) ou après l'étape d) ou après l'étape e).

5. Procédé d'isolement, selon l'une quelconque des revendications 1 à 4, **caractérisé en ce que** la saponine est dans un volume au moins égal ou supérieur au volume de l'échantillon à concentration égale.

6. Procédé d'isolement, selon l'une quelconque des revendications 1 à 5, **caractérisé en ce que** la concentration finale en saponine est supérieure à 0,02 % et inférieure ou égale à 20 %.

7. Procédé d'isolement, selon l'une quelconque des revendications 1 à 6, **caractérisé en ce que** la saponine est constituée par un triterpénoïde.

8. Procédé d'isolement, selon l'une quelconque des revendications 1 à 7, **caractérisé en ce que** l'enzyme apte à lyser les acides nucléiques libres est ensuite inactivée :
• chimiquement par l'ajout d'EDTA et/ou d'EGTA et/ou de DTT et/ou de β-mercaptoéthanol et/ou de DEPC et/ou de la guanidine
et/ou
• physiquement par l'augmentation de la température entre 40 et 100°C en présence ou non de détergents, tels que le Sodium Docécyl Sulfate (SDS).

9. Procédé d'isolement selon l'une quelconque des revendications 1 à 8, **caractérisé en ce que** lors du procédé, le pH est maintenu dans une fourchette entre 5 et 10, préférentiellement entre 6 et 9, par l'ajout d'une solution :
• basique si la valeur du pH est inférieure à 5, préférentiellement inférieure à 6,
• acide si la valeur du pH est supérieure à 10, préférentiellement supérieure à 9,
afin que le pH soit compris dans la fourchette.

10. Utilisation d'une formulation de saponine et d'une solution d'au moins une enzyme apte à lyser les acides nucléiques libres (ADN et/ou ARN) et d'au moins un agent de précipitation en solution, qui est du polyéthylène glycol, pour l'isolement sélectif de micro-organismes d'intérêt ou d'acides nucléiques de micro-organismes d'intérêt, dans un échantillon biologique liquide.

11. Utilisation selon la revendication 10, **caractérisée en ce qu'**elle utilise une formulation de saponine conduisant à une concentration finale supérieure à 0,02% et inférieure ou égale à 20%, et/ou un agent de précipitation à une concentration de 0,1 à 20%, et/ou une enzyme apte à lyser les acides nucléiques libres (ADN et/ou ARN) contenant entre 500 et 20000 unités enzymatiques.

12. Test de diagnostic basé sur un procédé selon l'une quelconque des revendications 1 à 9, ou sur une utilisation selon l'une quelconque des revendications 10 à 11.

13. Kit de diagnostic pour permettre la détection des micro-organismes d'intérêt et/ou la détection des acides nucléiques de micro-organismes d'intérêt au sein d'un échantillon biologique liquide comprenant ou susceptible de comprendre, notamment, des micro-organismes d'intérêt, des cellules non ciblées et, éventuellement, des virus à enveloppes, des mycoplasmes et/ou des débris de micro-organismes d'intérêt et/ou de cellules non ciblées, le kit comprenant:
(a) un récipient, et
(b) au moins une formulation de saponine,
(c) au moins une solution d'un agent de précipitation, qui est du polyéthylène glycol,
(d) au moins une enzyme apte à lyser les acides nucléiques, et
(e) le nécessaire pour détecter les micro-organismes d'intérêt et/ou les acides nucléiques de micro-organismes d'intérêt.

14. Kit, selon la revendication 13, **caractérisé par le fait que** le kit comprend en outre :
d') au moins une solution acide et/ou au moins une solution basique, et/ou
f) de l'EDTA et/ou de l'EGTA et/ou du DTT et/ou du β-mercaptoéthanol et/ou du DEPC et/ou de la guanidine et/ou
g) au moins un détergent ou un agent anionique.

## Patentansprüche

1. Verfahren zur selektiven Isolierung von interessierenden Mikroorganismen innerhalb einer flüssigen biologischen Probe, umfassend oder moglicherweise umfassend:
• interessierende Mikroorganismen, deren Zellmembran oder Kapsid kein Cholesterin enthalt, und
• Nicht-Ziel-Elemente, d. h.:
- Nicht-Zielzellen, deren Zellmembran Cholesterin enthalt, und
- moglicherweise umhüllte Viren, die Cholesterin enthalten, und
- moglicherweise enthalten, und Mykoplasmen, die Cholesterin,
- moglicherweise Trümmer von interessierenden Mikroorganismen und/oder Nicht Zielzellen,
wobei das Verfahren die folgenden Schritte umfasst:
a) Inkontaktbringen der flüssigen biologischen Probe mit einer Saponinformulierung, um Cholesterin enthaltende Zellmembranen oder Cholesterin enthaltende virale Hüllen oder Cholesterin enthaltende Mykoplasma- membranen zu destabilisieren,
b) Bewirken eines osmotischen Schocks von Nicht-Zielzellen, um sie spezifisch zu lysieren,
c) Zugabe eines Mittels zur Präzipitation der nicht-lysierten interessierenden Mikroorganismen in Lösung in der Probe, das Polyethylenglykol ist,
d) Zusetzen einer Lösung von mindestens einem Enzym, das zum Lysieren von freien Nukleinsäuren, DNA und/oder RNA, die aus den lysierten Nicht-Ziel- Elementen stammen, in Lösung in der Probe befähigt ist,
wodurch die gewünschten Mikroorganismen selektiv erhalten werden können.

2. Verfahren zum selektiven Isolieren von interessierenden Mikroorganismen aus einer flüssigen biologischen Probe gemäß Anspruch 1, wobei der Schritt des Zugebens eines Mittels zur Präzipitation der nicht-lysierten interessierenden Mikroorganismen in Losung in der Probe in Schritt a), nach den Schritten a) und b) oder nach Schritt c) durchgeführt wird.

3. Verfahren zum selektiven Isolieren von interessierenden Nukleinsäuren aus einer flüssigen biologischen Probe, umfassend oder moglicherweise umfassend:
• interessierende Mikroorganismen deren Zellmembran oder Kapsid kein Cholesterin enthalt, und
• Nicht-Ziel-Elemente, d. h.:
- Nicht-Zielzellen, deren Zellmembran Cholesterin enthalt, und
- moglicherweise umhüllte Viren, die Cholesterin enthalten, und
- moglicherweise enthalten, und Mykoplasmen, die Cholesterin
- moglicherweise Trümmer von interessierenden Mikroorganismen und/oder Nicht-Zielzellen,
wobei das Verfahren die folgenden Schritte umfasst:
a) Inkontaktbringen der flüssigen biologischen Probe mit einer Saponinformulierung, um Cholesterin enthaltende Zellmembranen und/oder Cholesterin enthaltende virale Hüllen und/oder Cholesterin enthaltende Mykoplasmamembranen zu destabilisieren,
b) Bewirken eines osmotischen Schocks von Nicht-Zielzellen, um sie spezifisch zu lysieren,
c) Zugabe eines Mittels zur Präzipitation der nicht-lysierten interessierenden Mikroorganismen in Lösung in der Probe, das Polyethylenglykol ist,
d) Zusetzen einer Lösung von mindestens einem Enzym, das zum Lysieren von freien Nukleinsäuren (DANN und/oder RNA), die aus den lysierten Nicht-Ziel-Elementen stammen, in Lösung in der Probe befähigt ist,
e) Inaktivieren des in Schritt (d) zugesetzten Enzyms und
f) Gewinnung der Nukleinsäuren der interessierten Mikroorganismen:
- nicht durch das Enzym aus Schritt (d) abgebaut und
- nicht durch die Wirkung der Schritte (a) und (b) zugänglich sind.

4. Verfahren zur selektiven Isolierung von interessierenden Nukleinsäuren in einer flüssigen biologischen Probe gemäß Anspruch 3, wobei der Schritt des Zugebens eines Mittels zur Präzipitation der nicht-lysierten interessierenden Mikroorganismen in Lösung in der Probe in Schritt a), nach den Schritten a) und b) oder nach Schritt c) oder nach Schritt d) durchgeführt wird.

5. Isolierungsverfahren gemäß einem der Ansprüche 1 bis 4, **dadurch gekennzeichnet, dass** das Saponin in einem Volumen vorliegt, das mindestens gleich oder großer als das Volumen der Probe gleicher Konzentration ist.

6. Isolierungsverfahren gemäß bis 5, **dadurch gekennzeichnet**, einem der Ansprüche 1 dass die Endkonzentration an Saponin großer als 0,02% und kleiner oder gleich 20% ist.

7. Isolierungsverfahren gemäß einem der Ansprüche 1 bis 6, **dadurch gekennzeichnet, dass** das Saponin aus einem Triterpenoid besteht.

8. Isolierungsverfahren gemäß einem der Ansprüche 1 bis 7, **dadurch gekennzeichnet, dass** das Enzym, das zum Lysieren der freien Nukleinsäuren befähigt ist, sodann inaktiviert wird auf:
• chemische Weise durch Zugabe von EDTA und/oder EGTA und/oder DTT und/oder beta-Mercaptoethanol und/oder DEPC und/oder Guanidin und/oder
• physikalische Weise durch Erhöhung der Temperatur zwischen 40 und 100°C in Gegenwart oder Abwesenheit von Detergenzien wie Natriumdocecylsulfat (SDS).

9. Verfahren nach einem der Ansprüche 1 bis 8, **dadurch gekennzeichnet, dass** der pH-Wert während des Verfahrens in einem Bereich zwischen 5 und 10, vorzugsweise zwischen 6 und 9 gehalten wird durch Zugabe einer:
• basischen Lösung, wenn der pH-Wert unter 5, vorzugsweise weniger als 6 ist,
• sauren Lösung, wenn der pH-Wert großer als 10, vorzugsweise großer als 9 ist,
so dass der pH-Wert in dem Bereich liegt.

10. Verwendung einer Saponin-Formulierung, einer Lösung von mindestens einem Enzym, das zum Lysieren von freien Nukleinsäuren, DNA und/oder RNA, befähigt ist und mindestens einem Präzipitationsmittel in Lösung in der Probe, das Polyethylenglykol ist, zur Isolierung von interessierenden Mikroorganismen oder Nuklein sauren von interessierenden Mikroorganismen in einer flüssigen biologischen Probe.

11. Verwendung gemäß Anspruch 10, **dadurch gekennzeichnet, dass** man eine Formulierung von Saponin, die zu einer Endkonzentration von mehr als 0,02% und weniger als oder gleich 20% führt, einem Präzipitationsmittel bei einer Konzentration von 0,1 bis 20% und einem Enzym, geeignet zum Lysieren freier Nukleinsäuren (DNA und/oder RNA) zwischen 500 und 20000 enzymatischen Einheiten, verwendet.

12. Diagnosetest, der auf einem Verfahren nach einem der Ansprüche 1 bis 9 oder auf einer geschätzten Verwendung nach einem der Ansprüche 10 bis 11 basiert.

13. Diagnose kit zum Ermöglichen der Erkennung von interessierenden Mikroorganismen und/oder ·der Erkennung von Nukleinsäuren der interessierenden Mikro-organismen innerhalb einer flüssigen biologischen Probe, umfassend oder moglicherweise umfassend interessierende Mikroorganismen, Nicht-Zielzellen und moglicherweise umhüllte Viren, Mykoplasmen und/oder Trümmer von interessierenden Mikroorganismen und/oder Nicht-Zielzellen, wobei das Kit Folgendes umfasst:
(a) einen Behälter und
(b) mindestens eine Saponinformulierung und
(c) mindestens eine Lösung von einem Präzipitationsmittel, das Polyethylenglykol ist,
(d) mindestens ein Enzym, Nukleinsäuren befähigt ist, und
(e) das Erforderliche für den Erkennung der Mikroorganismen von Interesse und/oder der Nukleinsäuren von Mikroorganismen von Interesse.

14. Kit gemäß Anspruch 13, dass das Kit weiterhin umfasst: **dadurch gekennzeichnet**:
d') mindestens eine saure Losung und/oder mindestens eine basische Losung und/oder
f) EDTA und/oder EGTA und/oder OTT und/oder beta-Mercaptoethanol und/oder DEPC und/oder Guanidin und/oder
g) mindestens ein Detergens oder ein anionisches Mittel.

## Claims

1. Method for selective isolation of microorganisms of interest in a liquid biological sample comprising or likely to comprise, notably:
• microorganisms of interest whose cell membrane or capsid does not contain cholesterol, and
• untargeted elements, i.e.:
- untargeted cells whose cell membrane contains cholesterol, and
- optionally, viruses with envelopes containing cholesterol, and
- optionally mycoplasmas containing cholesterol, and
- optionally, debris of microorganisms of interest and/or of untargeted cells,
said method comprising the following steps:
a) bringing the liquid biological sample into contact with a saponin formulation, in order to destabilize the cell membranes containing cholesterol or the viral envelopes containing cholesterol or the membranes of mycoplasmas containing cholesterol,
b) performing osmotic shock of the untargeted cells in order to lyse them specifically,
c) adding an agent for precipitating the unlysed microorganisms of interest in solution in the sample, which is polyethylene glycol,
d) adding a solution of at least one enzyme able to lyse the free nucleic acids (DNA and/or RNA) derived from the untargeted elements lysed in solution in the sample, allowing the microorganisms of interest to be obtained selectively.

2. Method for selective isolation, according to Claim 1, wherein the step consisting of adding an agent for precipitating the unlysed microorganisms of interest in solution in the sample is carried out in step a), after steps a) and b) or after step d).

3. Method for selective isolation of nucleic acids of interest in a liquid biological sample comprising or likely to comprise, notably:
• microorganisms of interest whose cell membrane or capsid does not contain cholesterol, and
• untargeted elements, i.e.:
- untargeted cells whose cell membrane contains cholesterol, and
- optionally, viruses with envelopes containing cholesterol, and
- optionally mycoplasmas containing cholesterol, and
- optionally, debris of microorganisms of interest and/or of untargeted cells,
said method comprising the following steps:
a) bringing the liquid biological sample into contact with a saponin formulation, in order to destabilize the cell membranes containing cholesterol and/or the viral envelopes containing cholesterol and/or the membranes of mycoplasmas containing cholesterol,
b) carrying out osmotic shock of the untargeted cells in order to lyse them specifically,
c) adding a precipitant of the unlysed microorganisms of interest in solution in the sample, which is polyethylene glycol,
d) adding a solution of at least one enzyme able to lyse the free nucleic acids (DNA and/or RNA) derived from the untargeted elements lysed in solution in the sample,
e) inactivating the enzyme added in step (d), and
f) making accessible the nucleic acids of microorganisms of interest:
- not degraded by the enzyme of step (d), and
- not accessible by the action of steps a) and b).

4. Method for selective isolation of nucleic acids of interest in a liquid biological sample, according to Claim 3, wherein the step consisting of adding an agent for precipitating the unlysed microorganisms of interest in solution in the sample is carried out in step a), after steps a) and b) or after step d) or after step e).

5. Method of isolation according to any one of Claims 1 to 4, **characterized in that** the saponin is in a volume at least greater than or equal to the volume of the sample at the same concentration.

6. Method of isolation according to any one of Claims 1 to 5, **characterized in that** the final saponin concentration is above 0.02% and less than or equal to 20%.

7. Method of isolation according to any one of Claims 1 to 6, **characterized in that** the saponin consists of a triterpenoid.

8. Method of isolation according to any one of Claims 1 to 7, **characterized in that** the enzyme able to lyse the free nucleic acids is then inactivated:
• chemically by adding EDTA and/or EGTA and/or DTT and/or β-mercaptoethanol and/or DEPC and/or guanidine
and/or
• physically by increasing the temperature between 40 and 100°C in the presence or in the absence of detergents, such as sodium dodecyl sulphate (SDS).

9. Method according to any one of Claims 1 to 8, **characterized in that** during the method, the pH is maintained in a range between 5 and 10, preferably between 6 and 9, by adding a solution that is:
• basic if the pH is below 5, preferably below 6,
• acidic if the pH is above 10, preferably above 9,
so that the pH is within the range.

10. Use of a saponin formulation and of a solution of at least one enzyme able to lyse the free nucleic acids (DNA and/or RNA) and of at least one precipitant in solution, which is polyethylene glycol, for isolation of microorganisms of interest or of nucleic acids of microorganisms of interest, in a liquid biological sample.

11. Use according to Claim 10, **characterized in that** it uses a saponin formulation leading to a final concentration above 0.02% and less than or equal to 20%, and/or a precipitant at a concentration from 0.1 to 20%, and/or an enzyme able to lyse the free nucleic acids (DNA and/or RNA) containing between 500 and 20000 enzyme units.

12. Diagnostic test based on a method according to any one of Claims 1 to 9, or on a use according to any one of Claims 10 to 11.

13. Diagnostic kit for isolating the microorganisms of interest and/or for isolating the nucleic acids of microorganisms of interest in a liquid biological sample comprising or likely to comprise, notably, microorganisms of interest, untargeted cells and, optionally, enveloped viruses, mycoplasmas and/or debris of microorganisms of interest and/or of untargeted cells, said kit comprising:
(a) a container, and
(b) at least one saponin formulation, and
(c) at least one solution of a precipitant, which is polyethylene glycol,
(d) at least one enzyme able to lyse the nucleic acids, and
(e) what is needed to detect the microorganisms of interest and/or the nucleic acids of microorganisms of interest.

14. Kit, according to Claim 13, **characterized in that** the kit further comprises:
d') at least one acid solution and/or at least one basic solution, and/or
f) EDTA and/or EGTA and/or DTT and/or β-mercaptoethanol and/or DEPC and/or guanidine and/or
g) at least one detergent or an anionic agent.
